# EUROPEAN PATENT SPECIFICATION

(11) **EP 2 779 945 B1**
(45) Date of publication and mention of the grant of the patent: **14.07.2021**
(21) Application number: 12794602.8
(22) Date of filing: 14.11.2012
(51) Int. Cl.: A61F 2/24, A61F 2/966

(54) **MEDICAL DEVICE WITH KEYED LOCKING STRUCTURES**
MEDIZINISCHE VORRICHTUNG MIT KORRESPONDIERENDEN SPERRSTRUKTUREN
DISPOSITIF MÉDICAL À STRUCTURES DE VERROUILLAGE CLAVETÉ

(30) Priority: 15.11.2011 US 201161559931 P
(43) Date of publication of application: 24.09.2014
(73) Proprietor: Boston Scientific Scimed, Inc., Maple Grove, MN 55311 (US)
(72) Inventor: SUTTON, Benjamin, San Jose, California 95119 (US); PAUL, David J., Scotts Valley, California 95066 (US); INO, Takashi, Los Gatos, CA 95032 (US)
(74) Representative: Peterreins Schley
(86) International application number: PCT/US2012/065076
(87) International publication number: WO 2013/074671

(56) References cited:
- US-A1- 2010 249 915
- US-A1- 2010 280 495
- US-A1- 2011 257 735
- None

## Description

### Technical Field

The present disclosure pertains to medical devices, and methods for manufacturing medical devices. More particularly, the present disclosure pertains to medical devices for delivering a replacement heart valve.

### Background

A wide variety of intracorporeal medical devices have been developed for medical use, for example, intravascular use. Some of these devices include guidewires, catheters, medical device delivery systems (e.g., for stents, grafts, replacement valves, etc.), and the like. These devices are manufactured by any one of a variety of different manufacturing methods and may be used according to any one of a variety of methods. Of the known medical devices and methods, each has certain advantages and disadvantages. There is an ongoing need to provide alternative medical devices as well as alternative methods for manufacturing and using medical devices.

US 2010/0280495 A1 discloses medical devices and delivery systems for delivering medical devices. The medical device can be locked in a locked configuration. US 2011/0257735 A1 discloses an apparatus for endovascularly delivering and releasing a prosthesis. The delivery system comprises a plurality of first actuatable elements adapted to engage a plurality of second elements.

### Brief Summary

The present invention pertains to a medical device delivery system as set forth in the appended claims. The invention provides design, material, manufacturing method, and use alternatives for medical devices including medical device delivery systems. The invention is directed to a medical device delivery system, which includes an outer sheath. An inner catheter is disposed within the outer sheath. An implant is releasably coupled to the inner catheter. The implant is configured to shift between a first elongated configuration and a second expanded configuration. The implant comprises a post and a buckle configured to lock the implant in the second expanded configuration, wherein the post and the buckle are attached to the implant. A push-pull rod for shifting the implant between the first configuration and the second configuration is coupled to the inner catheter and is configured to pull the post into engagement with the buckle. A locking assembly is disposed about the push-pull rod. The locking assembly has an interior passageway with a non-circular cross-sectional shape. The locking assembly is defined within the buckle coupled to the implant, or within a collar coupled to the inner catheter, or within a guide coupled to the inner catheter, or by a non-circular lumen formed within the inner catheter. At least a portion of an outer surface of the push-pull rod has a non-circular cross-sectional shape. The locking assembly has an interior passageway with a non-circular cross-sectional shape corresponding to the non-circular cross-sectional shape of the push-pull rod.

Also disclosed herein is an example medical device delivery system may include an outer sheath. An inner catheter may be disposed within the outer sheath. An implant may be releasably coupled to the inner catheter. The implant may be configured to shift between a first elongated configuration and a second expanded configuration. A push-pull rod for shifting the implant between the first configuration and the second configuration may be coupled to the inner catheter. A locking assembly may be disposed about the push-pull rod. At least a keyed portion of the push-pull rod may be keyed with a mating portion of the locking assembly so that the keyed portion of push-pull rod does not rotate relative to the locking assembly.

Also disclosed herein is an example method for shifting an implant from an elongated configuration to an expanded configuration may include providing a medical device delivery system. The medical device delivery system may include an outer sheath. An inner catheter may be disposed within the outer sheath. An implant may be releasably coupled to the inner catheter. The implant may be configured to shift between a first elongated configuration and a second expanded configuration. A push-pull rod for shifting the implant between the first configuration and the second configuration may be coupled to the inner catheter. A locking assembly may be disposed about the push-pull rod. At least a keyed portion of the push-pull rod may be keyed with a mating portion of the locking assembly so that the keyed portion of push-pull rod does not rotate relative to the locking assembly. The method may also include advancing the medical device to a position adjacent to an area of interest and proximally retracting the push-pull rod to shift the implant from the first configuration to the second configuration.

The above summary of some embodiments is not intended to describe each disclosed embodiment or every implementation of the present invention. The Figures, and Detailed Description, which follow, more particularly exemplify these embodiments.

### Brief Description of the Drawings

The invention may be more completely understood in consideration of the following detailed description of various embodiments of the invention in connection with the accompanying drawings, in which:
Figure 1 is side view of an example medical device system;
Figure 2 is a cross-sectional side view of an example outer sheath;
Figure 3 is a transverse cross-sectional view taken through line 3-3 in Figure 2;
Figure 4 is a side view of an example inner catheter;
Figure 5 is a cross-sectional view taken through line 5-5 in Figure 4;
Figure 6 is a cross-sectional view taken through line 6-6 in Figure 4;
Figure 7 is a perspective view of a portion of an example implant associated with the example medical device system;
Figures 8-11 are perspective views that illustrate an example mechanism for locking an implant;
Figure 12 is a side view of a portion of an example sheathing aid;
Figure 13 is an enlarged plan view illustrating engagement of the example sheathing aid with an example implant;
Figure 14 is a side view of an example handle;
Figure 15 is a cut away view illustrating some of the interior components of the example handle;
Figures 16-18 illustrate an example of coordinated movement of handle components within the example handle;
Figures 19-20 illustrate the rotation of a collar on the example handle;
Figures 21-22 illustrate some of the components within the example handle during rotation of the collar;
Figure 23 is a side view of a portion of another example device system;
Figure 24 is a cross-sectional view taken through line 24-24 in Figure 23;
Figure 25 is a cross-sectional view taken through line 25-25 in Figure 23;
Figure 26 is a cross-sectional view taken through line 26-26 in Figure 23;
Figure 27 is a cross-sectional view taken through line 27-27 in Figure 23;
Figure 28 is a cross-sectional view of a portion of an example medical device system;
Figure 29 is a cross-sectional view of an example push-pull rod;
Figure 30 is a cross-sectional view of another example push-pull rod;
Figure 31 is a cross-sectional view of another example push-pull rod;
Figure 32 is a side view of a portion of another example device system;
Figure 33 is a cross-sectional view taken through line 33-33 in Figure 32; and

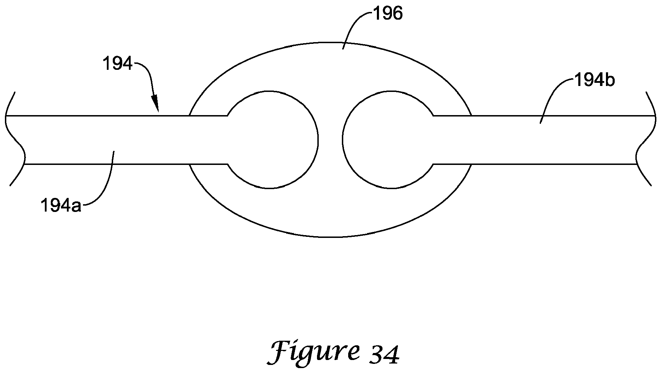

Figure 34 is a side view of another example push-pull rod.

While the invention is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the scope of the invention.

### Detailed Description

The present invention is defined in the appended claims.

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the term "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The drawings, which are not necessarily to scale, depict illustrative embodiments and are not intended to limit the scope of the invention.

Diseases and/or medical conditions that impact the cardiovascular system are prevalent in the United States and throughout the world. Traditionally, treatment of the cardiovascular system was often conducted by directly accessing the impacted part of the system. For example, treatment of a blockage in one or more of the coronary arteries was traditionally treated using coronary artery bypass surgery. As can be readily appreciated, such therapies are rather invasive to the patient and require significant recovery times and/or treatments. More recently, less invasive therapies have been developed, for example, where a blocked coronary artery could be accessed and treated via a percutaneous catheter (e.g., angioplasty). Such therapies have gained wide acceptance among patients and clinicians.

Some relatively common medical conditions may include or be the result of inefficiency, ineffectiveness, or complete failure of one or more of the valves within the heart. For example, failure of the aortic valve can have a serious effect on a human and could lead to serious health condition and/or death if not dealt with. Treatment of defective heart valves poses other challenges in that the treatment often requires the repair or outright replacement of the defective valve. Such therapies may be highly invasive to the patient. Disclosed herein are medical devices that may be used for delivering a medical device to a portion of the cardiovascular system in order to diagnose, treat, and/or repair the system. At least some of the medical devices disclosed herein may be used to deliver and implant a replacement heart valve (e.g., a replacement aortic valve). In addition, the devices disclosed herein may deliver the replacement heart valve percutaneously and, thus, may be much less invasive to the patient. The devices disclosed herein may also provide a number of additional desirable features and benefits as described in more detail below.

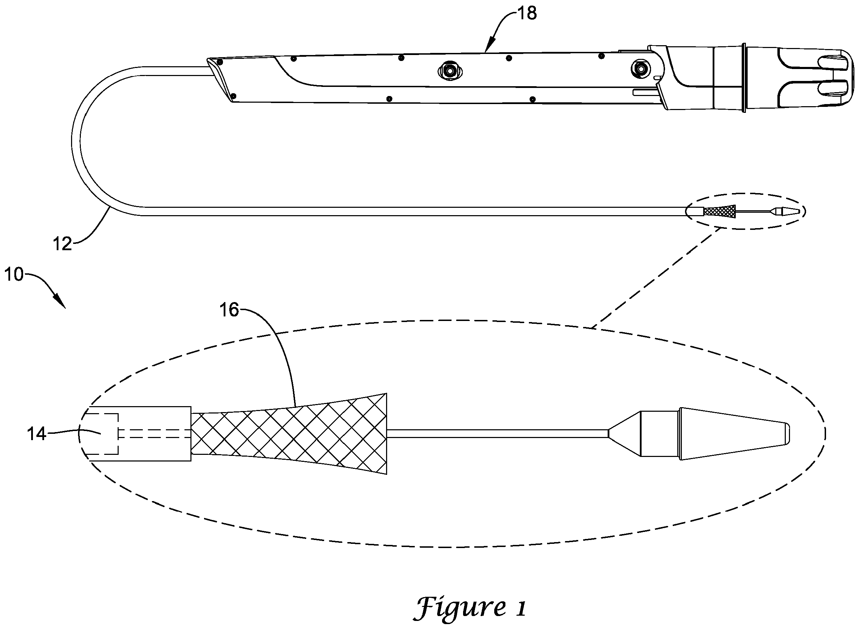

Figure 1 is a side view of an example medical device system 10. It should be noted that some features of system 10 are either not shown, or are shown schematically, in Figure 1 for simplicity. Additional details regarding some of the components of system 10 are provided in other figures in greater detail. System 10 may be used to deliver and/or deploy a variety of medical devices to a number of locations within the anatomy. In at least some embodiments, system 10 is a replacement heart valve delivery system (e.g., a replacement aortic valve delivery system) that can be used for percutaneous delivery of a replacement heart valve. This, however, is not intended to be limiting as system 10 may also be used for other interventions including mitral valve replacement, valve repair, valvuloplasty, and the like, or other similar interventions.

System 10 may generally be described as a catheter system that includes an outer sheath or catheter 12 and an inner catheter or tube 14 (a portion of which is shown in Figure 1 in phantom line) extending at least partially through outer sheath 12. A medical device implant 16 may be coupled to inner catheter 14 and disposed within outer sheath 12 during delivery of implant 16. A handle 18 may be disposed at the proximal end of outer sheath 12 and inner catheter 14. In general, handle 18 may be configured to manipulate the position of outer sheath 12 relative to inner catheter 14 as well as aid in the deployment of implant 16.

In use, system 10 may be advanced percutaneously through the vasculature to a position adjacent to an area of interest. For example, system 10 may be advanced through the vasculature to a position adjacent to a defective aortic valve. During delivery, implant 16 may be generally disposed in an elongated and low profile "delivery" configuration within outer sheath 12. Once positioned, outer sheath 12 may be retracted to expose implant 16. Implant 16 may be actuated in order to expand implant into a generally shortened and larger profile "deployed" configuration suitable for implantation within the anatomy. When implant 16 is suitably deployed within the anatomy, system 10 can be removed from the vasculature, leaving implant 16 in place to function as, for example, a suitable replacement for the native aortic valve. In at least some interventions, implant 16 may be deployed within the native valve (e.g., the native valve is left in place and not excised). Alternatively, the native valve may be removed and implant 16 may be deployed in its place as a replacement.

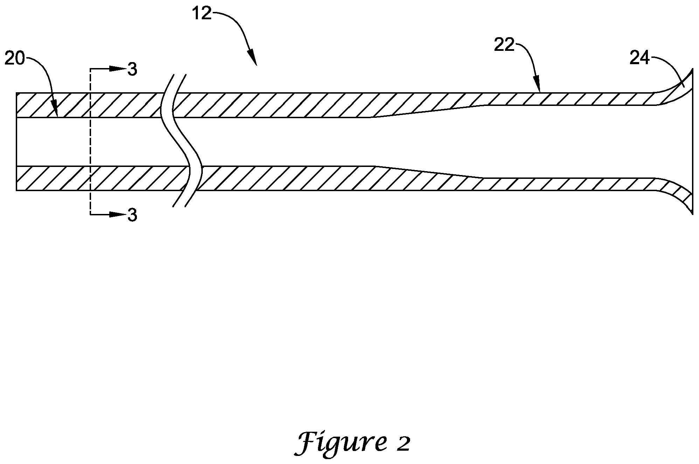

Figures 2-13 (as well as other figures) illustrate some of the components of system 10. For example, Figure 2 is a cross-sectional side view of outer sheath 12. Here it can be seen that outer sheath 12 has a proximal portion 20 and a distal portion 22. Distal portion 22 may have a slightly enlarged or flared inner diameter, which may provide additional space for holding implant 16 therein. For example, the inner diameter of outer sheath 12 along proximal portion 20 may be in the range of about 0.254 to 1.27 cm (0.10 to 0.50 inches), or about 0.508 to 1.016 cm (0.20 to 0.40 inches), or about 0.508 to 0.762 cm (0.20 to 0.30 inches), or about 0.56388 ± 0.0508 cm (0.222 ± 0.002 inches). The inner diameter of outer sheath 12 along distal portion 22 may be in the range of about 0.254 to 1.27 cm (0.10 to 0.50 inches), or about 0.508 to 1.016 cm (0.20 to 0.40 inches), or about 0.508 to 0.762 cm (0.20 to 0.30 inches), or about 0.579 to 0.5842 cm (0.228 to 0.230 inches). At the distal end of distal portion 22 may be a distal tip 24, which may be flared or otherwise have a funnel-like shape. The funnel-like shape increases the outer diameter (and inner diameter) of outer sheath 12 at distal tip 24 and may aid in the sheathing and/or resheathing of implant 16 into outer sheath 12. Other than at distal tip 24, outer sheath 12 may have a generally constant outer diameter. For example, outer sheath 12 may have an outer diameter in the range of about 0.254 to 1.27 cm (0.10 to 0.50 inches), or about 0.508 to 1.016 cm (0.20 to 0.40 inches), or about 0.508 to 0.762 cm (0.20 to 0.30 inches), or about 0.6858 cm (0.270 inches). These are just examples. Other embodiments are contemplated that have differing dimensions (including those appropriate for differently sized patients including children) and/or arrangements for the outer diameter and/or inner diameter of outer sheath 12. These contemplated embodiments include outer sheaths with flared or otherwise variable outer diameters, embodiments with constant inner diameters, combinations thereof, and the like. Outer sheath 12 may also have a length that is appropriate for reaching the intended area of interest within the anatomy. For example, outer sheath 12 may have a length in the range of about 30 to 200 cm, or about 60 to 150 cm, or about 100 to 120 cm, or about 108 ± 0.20 cm. Outer sheath 12 may also be curved. For example, a distal section of outer sheath 12 may be curved. In one example, the radius of the curve (measured from the center of outer sheath 12) may be in the range of about 2 to 6 cm (20 to 60 mm), or about 3 to 4 cm (30 to 40 mm), or about 3.675 cm (36.75 mm). Again, these dimensions are examples and are not intended to be limiting.

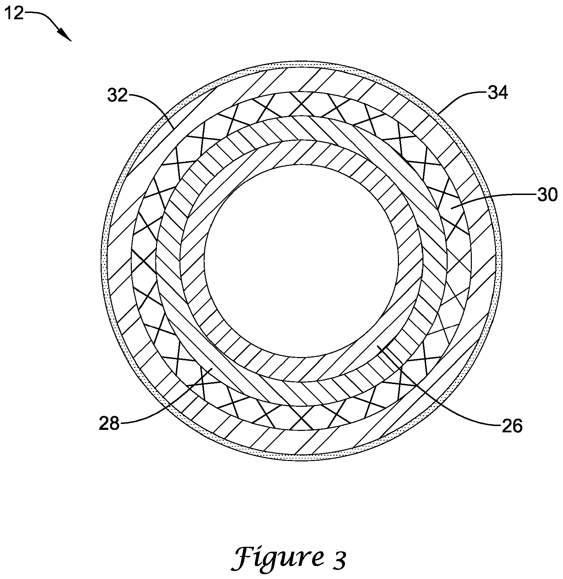

Outer sheath 12 may be formed from a singular monolithic tube or unitary member. Alternatively, outer sheath 12 may include a plurality of layers or portions. One or more of these layers may include a reinforcing structure such as a braid, coil, mesh, combinations thereof, or the like. Figure 3 illustrates one example of a multilayer structure for outer sheath 12. For example, outer sheath 12 may include an inner liner or layer 26. An intermediate or tier layer 28 may be disposed on inner liner 26. A reinforcement 30 may be disposed on intermediate layer 28. A topcoat or outer layer 32 may be disposed on reinforcement 30. Finally, an outer coating 34 (e.g., a lubricious coating, a hydrophilic coating, a hydrophobic coating, etc.) may be disposed along portions or all of topcoat 32. These are just examples. Several alternative structural configurations are contemplated for outer sheath 12 including embodiments including two or more layers that may be different from those shown in Figure 3, embodiments without a reinforcement, and the like, or other suitable configurations.

The dimensions and materials utilized for the various layers of outer sheath 12 may also vary. For example, inner liner 26 may include a polymeric material such as fluorinated ethylene propylene (FEP) and may have a thickness in the range of about 0.00254 to 0.0127 cm (0.001 to 0.005 inches) or about 0.00762 ± 0.00254 (0.003 ± 0.001 inches), intermediate layer 28 may include a polymer material such as polyether block amide (e.g., PEBAX 6333) and may have a thickness in the range of about 0.00254 to 0.0127 cm (0.001 to 0.005 inches) or about 0.00508 ± 0.00254 (0.002 ± 0.001 inches), outer coating 34 may include a polymer material such as polyether block amide (e.g., PEBAX 7233) and may have a thickness in the range of about 0.00254 to 0.0254 cm (0.001 to 0.01 inches). In some embodiments, outer coating 34 may vary in thickness. For example, along proximal portion 20 outer coating 34 may have greater thickness, such as about 0.0127 to about 0.0508 cm or about 0.02159 cm (0.005 to 0.02 inches or about 0.0085 inches), than along distal portion 22 and/or distal tip 24, which may be about 0.0127 to about 0.0508 cm or about 0.01651 cm (e.g., about 0.005 to 0.02 inches or about 0.0065 inches). These are just examples as other suitable materials may be used.

The form of distal tip 24 may also vary. For example, in at least some embodiments, inner liner 26 (i.e., a 2.5 mm section thereof) may be extended up and around the distal end of outer sheath 12 (e.g., around reinforcement 30 and topcoat 32). A ring member (not shown) made from a suitable material such as a 55D polyether block amide (e.g., 55D PEBAX) may be disposed over inner liner 26 and heat bonded to form distal tip 24. This may form the funnel-like shape of distal tip 24.

Reinforcement 30 may also vary in form. In at least some embodiments, reinforcement 30 may take the form of a braid, coil, mesh, or the like. For example, in some embodiments, reinforcement 30 may include a metallic braid (e.g., stainless steel). In some of these embodiments, reinforcement 30 may also include additional structures such as one or more longitudinally-extending strands. For example, reinforcement 30 may include a pair of longitudinally-extending aramid and/or para aramid strands (for example, KEVLAR®) disposed on opposite sides of the braid. These strands may or may not be woven into portions or all of the braid.

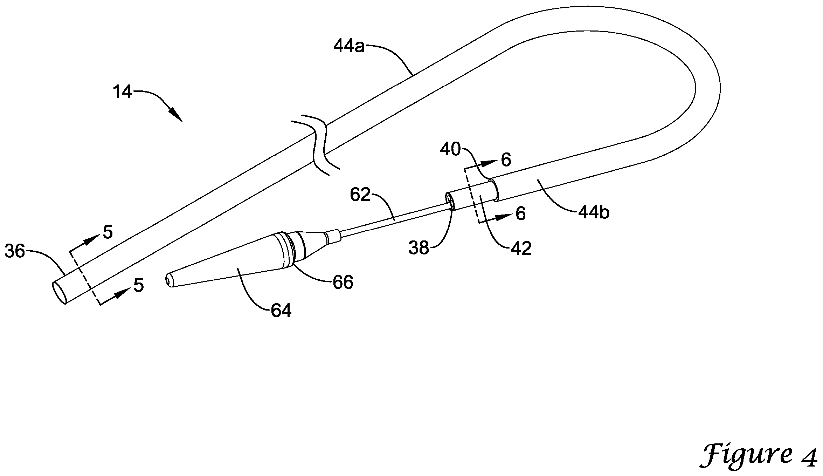

Figure 4 is a side view of the inner catheter 14. A distal end region of inner catheter 14 may include a step in outer diameter 40 that defines a decreased outer diameter section 42. For example, decreased outer diameter section 42 may have an outer diameter in the range of about 0.127 to 0.635 cm (0.05 to 0.25 inches), or about 0.254 to 0.508 cm (0.10 to 0.20 inches), or about 0.38608 ± 0.00762 (0.152 ± 0.003 inches) as opposed to the remainder of inner catheter 14 where the outer diameter may be in the range of about 0.127 to 0.762 cm (0.05 to 0.30 inches), or about 0.254 to 0.635 cm (0.10 to 0.25 inches), or about 0.508 ± 0.0254 cm (0.20 ± 0.01 inches). Decreased outer diameter section 42 may define a region where other components of system 10 may be attached. Some additional details regarding these components can be found herein.

In general, inner catheter 14 may take the form of an extruded polymer tube. Other forms are also contemplated including other polymer tubes, metallic tubes, reinforced tubes, or the like including other suitable materials such as those disclosed herein. In some embodiments, inner catheter 14 is a singular monolithic or unitary member. In other embodiments, inner catheter 14 may include a plurality of portions or segments that are coupled together. The total length of inner catheter may be in the range of about 60 to 150 cm, or about 80 to 120 cm, or about 100 to 115 cm, or about 112 ± 0.02 cm. Just like outer sheath 12, inner catheter 14 may also be curved, for example adjacent to the distal end thereof. In some embodiments, inner catheter 14 may have one or more sections with a differing hardness/stiffness (e.g., differing shore durometer). For example, inner catheter may have a proximal region 44a and an intermediate region 44b. Proximal region 44a may include a generally stiff polymeric material such as a 72D polyether block amide (e.g., 72D PEBAX) and may have a length in the range of about 60 to 150 cm, or about 80 to 120 cm, or about 100 to 115 cm, or about 109.5 ± 0.02 cm. Intermediate region 44b may include a 40D polyether block amide (e.g., 40D PEBAX) and may have a length in the range of about 5 to 25 mm, or about 10 to 20 mm, or about 15 ± 0.01 mm. Section 42 may also differ from regions 44a/44b and, in some embodiments, may include a 72D polyether block amide (e.g., 72D PEBAX) and may have a length in the range of about 0.5 to 2 cm (5 to 20 mm), or about 0.8 to 1.5 cm (8 to 15 mm), or about 1 ± 0.001 cm (10 ± 0.01 mm). These are just examples.

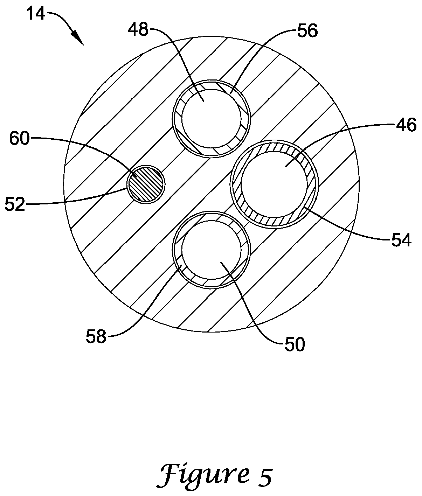

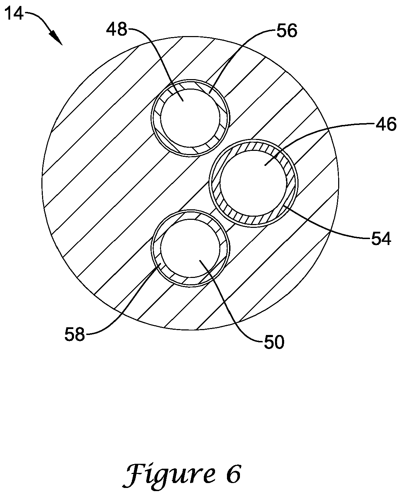

Inner catheter 14 may include one or more lumens. For example, Figure 5 (which is a cross sectional view of inner catheter 14 adjacent to proximal end portion 36) illustrates that inner catheter 14 may include a first lumen 46, a second lumen 48, a third lumen 50, and a fourth lumen 52. In general, lumens 46/48/50/52 extend along the entire length of inner catheter 14. Other embodiments are contemplated, however, where one or more of lumens 46/48/50/52 extend along only a portion of the length of inner catheter 14. For example, fourth lumen 52 may stop just short of the distal end of inner catheter 14 and/or be filled in at its distal end to effectively end fourth lumen 52 proximal of the distal end of inner catheter 14, as illustrated in Figure 6 by the absence of fourth lumen 52 adjacent to the distal end of inner catheter 14.

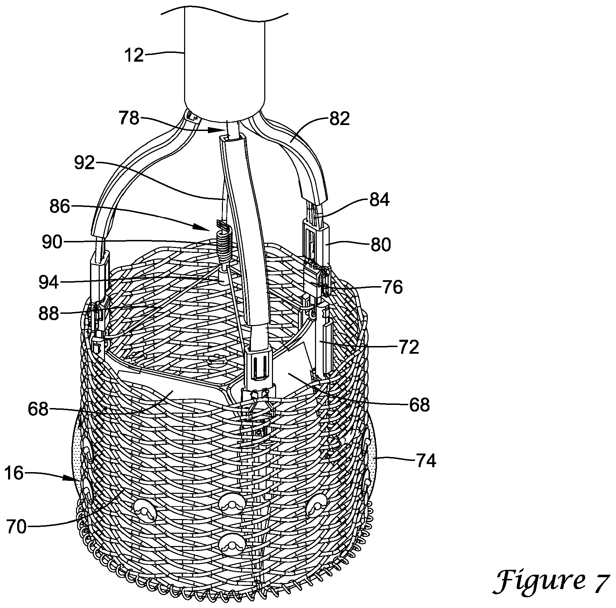

Disposed within first lumen 46 may be push-pull rods 84 (not shown in Figure 5, seen in other figures including Figure 7), which are used to expand and/or elongate implant 16 as explained in more detail herein. In at least some embodiments, first lumen 46 may be lined with a low friction liner 54 (e.g., a FEP liner). Disposed within second lumen 48 may be a pin release mandrel 92 (not shown in Figure 5, seen in other figures including Figure 7), which is also explained in more detail herein. In at least some embodiments, second lumen 48 may be lined with a hypotube liner 56. Third lumen 50 may be a guidewire lumen and this lumen may also be lined with a hypotube liner 58.

Fourth lumen 52 may be used to house a non-stretch wire 60. The form of non-stretch wire 60 may vary. In some embodiments, non-stretch wire 60 may take the form of a stainless steel braid. The non-stretch wire 60 may optionally include a pair of longitudinally-extending aramid and/or para aramid strands (for example, KEVLAR®) disposed on opposite sides of the braid. In general, rather than being "disposed within" fourth lumen 52, non-stretch wire 60 may be embedded within fourth lumen 52. In addition, non-stretch wire 60 may extend to a position adjacent to distal end portion 38 but not fully to the distal end of inner catheter 14 as illustrated in Figure 6 by the absence of fourth lumen 52 adjacent to the distal end of inner catheter 14. For example, a short distal segment of fourth lumen 52 may be filled in with polymer material adjacent to the distal end of inner catheter 14.

Inner catheter 14 may also include a guidewire tube extension 62 that extends distally from distal end portion 38. A nose cone 64 is attached to guidewire tube extension 62. Nose cone 64 generally is designed to have an atraumatic shape. Nose cone 64 may also include a ridge or ledge 66 that is configured to abut the distal tip 24 of outer sheath 12 during delivery of implant 16.

Figure 7 illustrates some of the additional components of system 10 and implant 16. For example, here it can be seen that implant 16 includes a plurality of valve leaflets 68 (e.g., bovine pericardial) which are secured to a cylindrical braid 70 at a post or commissure post 72, for example at the commissure portions of the leaflets 68. In this example, implant 16 includes three leaflets 68 secured to braid 70 with three posts 72. Leaflets 68 may also be secured to the base or "distal end" of braid 70. The posts 72, in turn, may be secured to braid 70 (e.g., along the interior of braid 70) with sutures or other suitable mechanisms. Positioned adjacent to (e.g., longitudinally spaced from and aligned with) posts 72 are a plurality of buckles 76, which may also be sutured to braid 70 (e.g., along the interior of braid 70). In this example, one buckle 76 is attached to braid 70 adjacent to each of the three posts 72. Accordingly, braid 70 has a total of three buckles 76 and three posts 72 attached thereto. Other embodiments are contemplated where fewer or more buckles 76 and posts 72 may be utilized. A seal 74 (shown in cross-section) may be disposed about braid 70 and, as the name suggests, may help to seal implant 16 within a target implant site or area of interest.

Attachment between implant 16 and inner catheter 14 (and/or outer sheath 12) may be effected through the use of a three finger coupler 78. Coupler 78 may generally include a cylindrical base (not shown) that is attached to inner catheter 14 (e.g., disposed about and attached to reduced outer diameter section 42). Projecting distally from the base are three fingers that are each configured to engage with implant 16 at posts 72 and buckles 76. A collar 80 may further assist in holding together these structures. A guide 82 may be disposed over each of the fingers and may serve to keep the fingers of coupler 78 associated with push-pull rods 84 extending adjacent to coupler 78. Finally, a pin release assembly 86 may be a linking structure that keeps posts 72, buckles 76, and push-pull rods 84 associated with one another. Pin release assembly 86 includes a plurality of individual pins 88 that may be joined together via a coiled connection 90 and held to a pin release mandrel 92 with a ferrule 94.

During delivery, implant 16 is secured at the distal end of inner catheter 14 by virtue of the association of the fingers of coupler 78 being coupled with a projecting proximal end of buckles 76 (and being held in place with collar 80 disposed over the connection) and by virtue of pins 88 securing together push-pull rods 84 and posts 72. When implant 16 is advanced within the anatomy to the desired location, outer sheath 12 may be withdrawn (e.g., moved proximally relative to inner catheter 14) to expose implant 16. Then, push-pull rods 84 can be used to expand and "lock" implant 16 in the expanded or deployed configuration by proximally retracting push-pull rods 84 to pull posts 72 into engagement with buckles. Finally, pins 88 can be removed, thereby uncoupling push-pull rods 84 from posts 72, which allows implant 16 to be released from system 10 and deployed in the anatomy.

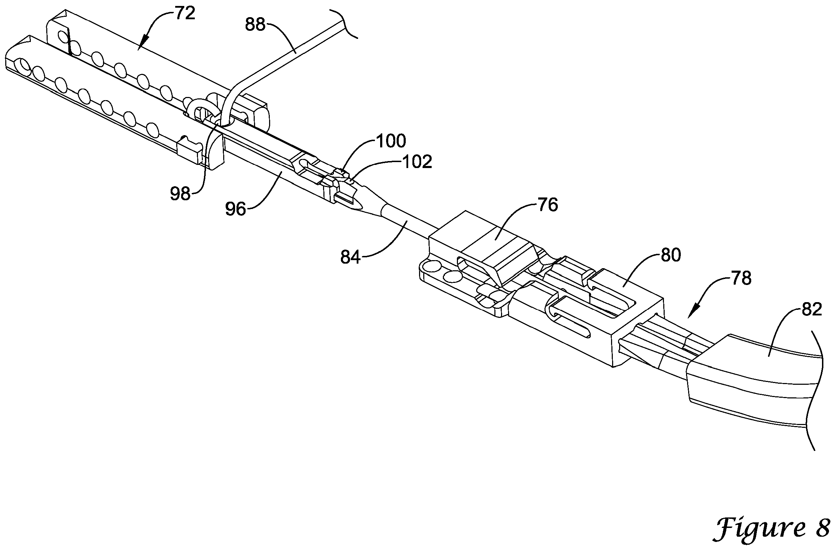

Figures 8-11 illustrate the locking system utilized with system 10. For simplicity purposes, only one of the three fingers of the coupler 78, only one of the three push-pull rods 84, and only one of the posts 72 of the example system 10 are shown (and implant 16 is not shown). As seen in Figure 8, push-pull rod 84 extends through guide 82 adjacent to the fingers of coupler 78, through collar 80, through buckle 76, and into a hollow t-shaped bar portion 96 of post 72. The distal end of push-pull rod 84 may include an opening or aperture (not shown) that can be aligned with an opening 98 of t-shaped bar portion 96. When so aligned, pin 88 can be looped through opening 98 and the opening of push-pull rod 84. This secures push-pull rod 84 to post 72 and forms a configuration of these structures that can be utilized during delivery of implant 16. As can be appreciated, the proximal end of post 72 and the distal end of buckle 76 are longitudinally separated and, accordingly, implant 16 is in an elongated and generally low-profile configuration suitable for delivery.

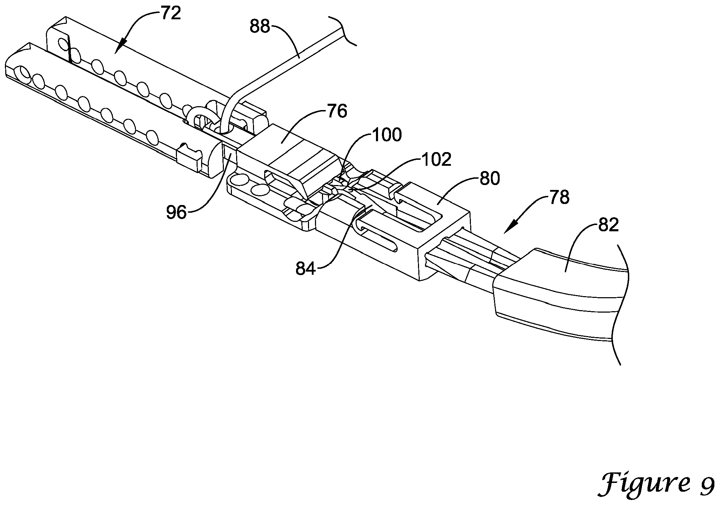

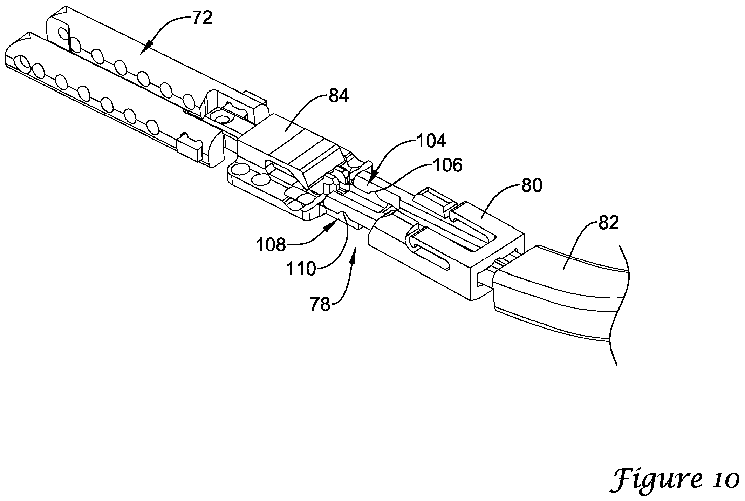

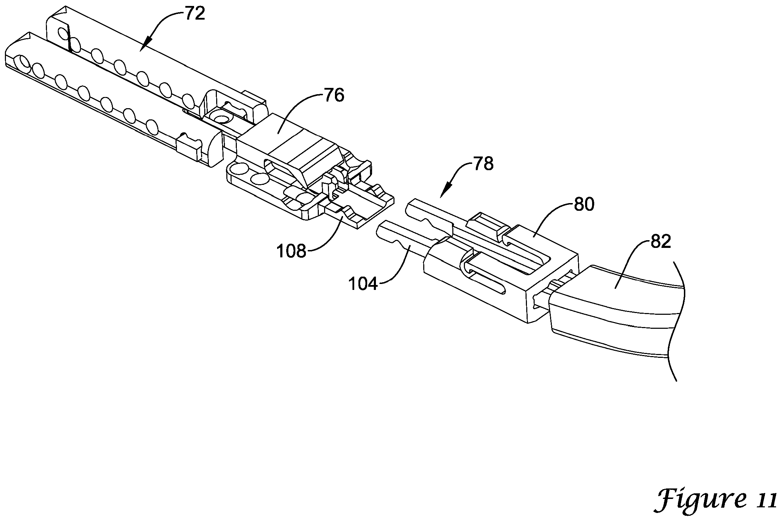

When implant 16 reaches the intended target site within the anatomy, a clinician can proximally retract push-pull rod 84, thereby moving the proximal ends of posts 72 toward the distal ends of buckles 76 in order to expand implant 16. Ultimately, push-pull rod 84 can be retracted sufficiently far enough to lock post 72 with buckle 76 so as to lock implant in an expanded configuration suitable for implantation within the anatomy. Figure 9 illustrates push-pull rod 84 proximally retracted. In doing so, post 72 is brought into contact with buckle 76. More particularly, a raised, generally transversely-oriented ridge 100 on t-shaped bar portion 96 may be pulled proximally past buckle 76 so that post 72 is secured and held in place by buckle 76. At this point, it is possible to urge push-pull rods 84 distally to "unlock" implant 16, thereby allowing for repositioning and/or retraction. Alternatively, if a clinician is satisfied with the positioning and/or locking of implant 16 (e.g., after visualization of implant 16 via a suitable imaging technique), pins 88 may be pulled (e.g., removed from openings 98 and the openings in push-pull rods 84) to uncouple push-pull rods 84 from posts 72 as shown in Figure 10. Further retraction of push-pull rods 84 causes a longitudinally-oriented ridge 102 on push-pull rods 84 to engage collar 80 and causes collar 80 to slide proximally along the fingers of coupler 78. In doing so, a forked end 104 of the fingers, which has a groove 106 formed therein, is exposed and can be uncoupled from a rail 108, which has a projection 110 formed thereon that is configured to mate with groove 106, as shown in Figure 11. Thereafter, system 10 can be removed from the anatomy, leaving behind the expanded and deployed implant 16.

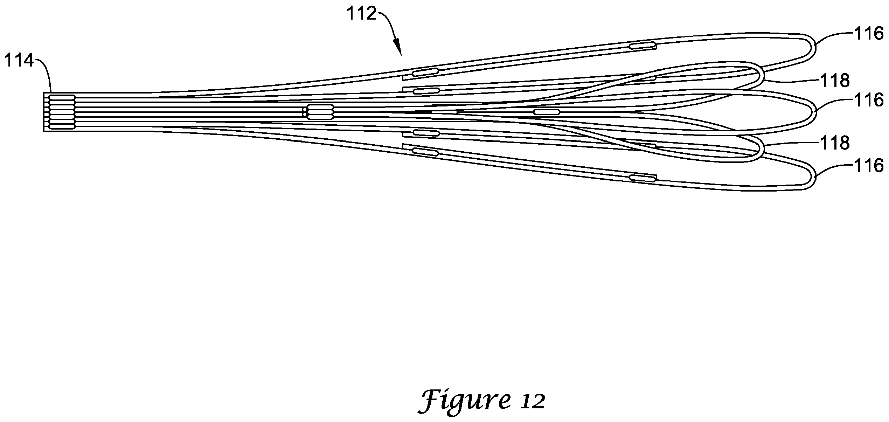

Figures 12-13 illustrate another component that may be included with system 10. For example, Figure 12 is a side view of a portion of a sheathing aid 112. Here it can be seen that sheathing aid 112 includes a base 114 and a group of petals including a set of three longer petals 116 and a pair of shorter petals 118. In use, a group of petals 116/118 may be positioned between each of the fingers of coupler 78. Because the coupler 78 may have a total of three fingers, sheathing aid 112 may have a total of fifteen petals (e.g., three groups that each include three "long" petals 116 and two "short" petals 118, with each group being positioned between adjacent pairs of fingers of coupler 78). Base 114 may be secured to inner catheter 14 adjacent to coupler 78 (e.g., underneath coupler 78 and between coupler 78 and inner catheter 14).

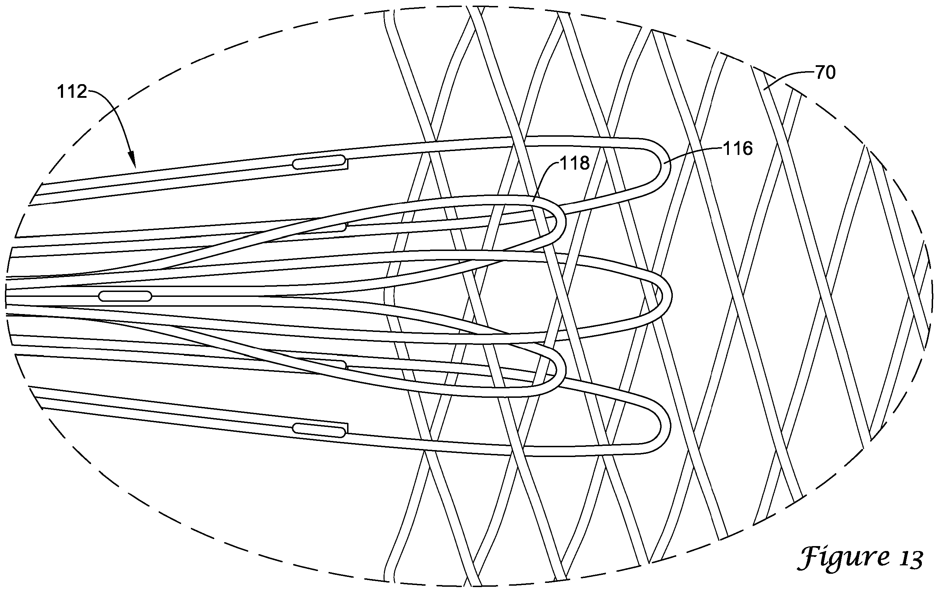

Sheathing aid 112, as the name suggests, may be used to aid in the sheathing of implant 16 into outer sheath 12. In addition, sheathing aid 112 may aid in the initial sheathing of implant 16 (e.g., removing implant 16 from a packaging container such as a bottle and pulling implant 16 into outer sheath 12) and in re-sheathing implant 16 during repositioning and/or retraction of implant 16 within the area of interest. Sheathing may be accomplished via the arrangement and positioning of the various petals 116/118. For example, Figure 13 illustrates the longer petals 116 woven in and out of braid 70, and the shorter petals 118 disposed along the exterior of braid 70 acting as a funnel for sheathing.

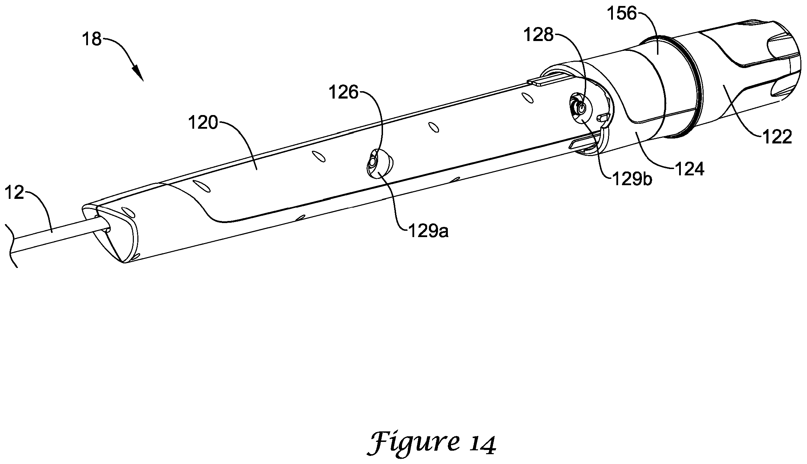

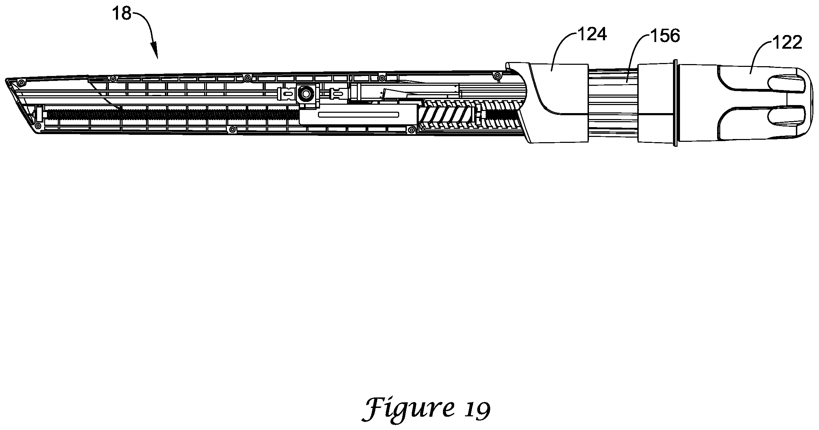

Figure 14 is a side view of handle 18. Here it can be seen that handle 18 includes a handle housing 120. A rotatable control knob 122 may be disposed about handle housing 120 (e.g., at a proximal end of handle housing 120) and may be used to move one or more of the components of system 10 (e.g., outer sheath 12, push-pull rods 84, etc.). A rotatable collar 156 may be disposed about the handle housing 120. Control knob 122 may be disposed about a proximal portion of collar 156. A slidable door 124 may also be disposed about handle housing 120. Door 124 may translate distally to expose a distal portion of rotatable collar 156 (not shown in Figure 14, can be seen in other figures including Figures 19-20) positioned generally under door 124. Collar 156 may be rotated to move one or more components of system 10 (e.g., push-pull rods 84, pin release mandrel 92, etc.). Handle 18 may also include one or more apertures 129a/129b and/or flush ports 126/128 that can be used to flush system 10. In some embodiments, distal flush port 126 and proximal flush port 128 may be accessible from the exterior of the handle housing 120 through distal aperture 129a and proximal aperture 129b, respectively.

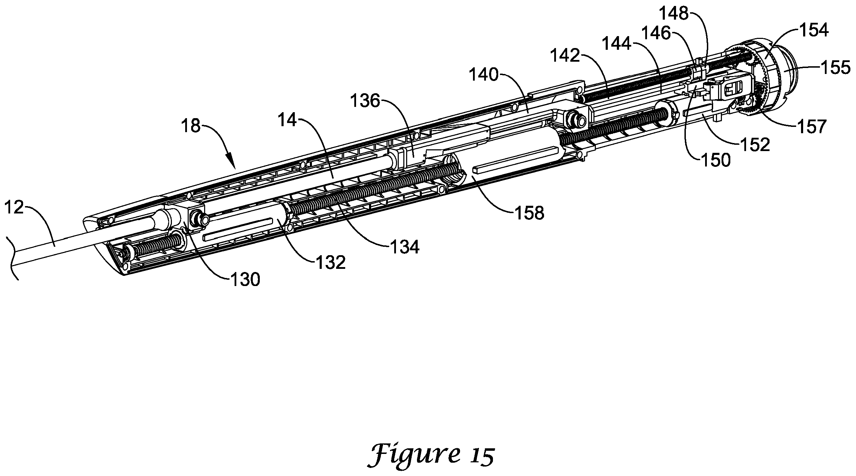

Figure 15 is a side view of handle 18 with a portion of handle housing 120 removed, exposing at least some of the interior components. Here it can be seen that outer sheath 12 may be attached to a sheath adapter 130. Sheath adapter 130 is attached to a sheath carriage 132, which may be threaded onto a lead screw 134. Distal flush port 126 may be disposed on sheath adapter 130. In general, distal flush port 126 provides access to the interior or lumen of outer sheath 12 (e.g., access to space between inner catheter 14 and outer sheath 12) so that a clinician can flush fluid through the lumen of outer sheath 12 to remove any unwanted materials (e.g., air, fluid, contaminants, etc.) therein prior to use of system 10. In at least some embodiments, distal flush port 126 has a luer type connector (e.g., a one-way luer connector) that allows a device such as a syringe with a corresponding connector to be attached thereto for flushing.

Extending through and proximally from sheath adapter 130 is inner catheter 14. A proximal end of inner catheter 14 is attached (e.g., fixedly attached) to an interior body or diverter 136. Diverter 136 is attached to a support body 140. In general, diverter 136 and/or support body 140 may have one or more passageways or lumens formed therein. In some embodiments, push-pull rods 84 and/or pin release mandrel 92 may extend through respective passageways. Alternatively, the proximal ends of push-pull rods 84 and/or pin release mandrel 92 may each be attached to a shaft or hypotube (e.g., solid in cross-section, tubular, etc.), and each of the shafts may extend through the one or more passageways. For example, a first shaft or hypotube 142 and a second shaft or hypotube 144 may extend through the passageways in diverter 136, and in some embodiments, the first shaft or hypotube 142 extends through a first passageway and the second shaft or hypotube 144 extends through a second passageway that is separate or distinct from the first passageway. In at least some embodiments, first shaft 142 is attached to pin release mandrel 92. In at least some embodiments, second shaft 144 is attached to push-pull rods 84. It should be noted that at in least some embodiments of system 10, three push-pull rods 84 are utilized. In these embodiments, the three push-pull rods 84 come together (e.g., brought into contact with one another or otherwise brought into relatively close proximity with one another) adjacent to the distal end of inner catheter 14 and enter first lumen 46. At one or more positions along their length, push-pull rods 84 may be attached to one another. For example, in some embodiments, push-pull rods 84 may be welded together about 10.16 cm (about 4.00 inches) from their distal ends. In some embodiments, push-pull rods 84 may be welded together proximate their proximal ends in addition to or instead of the distal weld. Proximally thereafter, push-pull rods 84 may extend to second shaft 144.

A hypotube (e.g., hypotube liner 58 disposed along guidewire lumen 52) may extend through diverter 136 within a passageway therein and then be "diverted" around a portion of diverter 136 and support body 140, and ultimately be extended to a position at the proximal end of handle 18 so as to provide a user access to guidewire lumen 52. Proximal flush port 128 may be disposed on support body 140 that can be used to flush the lumens of inner catheter 14 and, for example, may function similarly to distal flush port 126.

At their respective proximal ends, first shaft 142 may be secured to a slider 146 and second shaft 144 may be secured to a force limiter body 150. The connections between the various components may include a number of different types of connections including mechanical bonding (e.g., pinning, threading, interference fit, etc.), adhesive bonding, thermal bonding, etc. Slider 146 may be slidable relative to force limiter body 150. In some embodiments, slider 146 may be selectively locked to force limiter body 150, thereby preventing relative movement between the slider 146 and the force limiter body 150. Force limiter body 150 may be secured to a push-pull rod carriage 152, which may be threaded onto lead screw 134. Thus, movement of lead screw 134 can cause movement of push-pull rod carriage 152 and force limiter body 150 and thus, push-pull rods 84 (via second shaft 144). Some additional details regarding this motion can be found herein.

In general, force limiter body 150 forms or defines a stop point that provides tactile feedback (e.g., resistance to further rotation of control knob 122) to the user indicating that push-pull rods 84 have been retracted proximally a sufficient distance to lock posts 72 with buckles 76. To verify proper locking, a clinician may use an appropriate visualization technique to visualize proper locking (e.g., the relative positioning of the posts 72 and the buckles 76). A chock 148 may be positioned adjacent to slider 146 to selectively lock slider 146 to force limiter body 150. In order to allow pin release mandrel 92 to be proximally retracted to pull pins 88, chock 148 can be rotated or otherwise moved to a secondary position or configuration. When in this configuration, chock 148 no longer forms a barrier to further movement of, for example, slider 146 and pin release mandrel 92. Accordingly, with chock 148 no longer acting as an impediment, slider 146 and pin release mandrel 92 can be proximally retracted to facilitate deployment of implant 16 by allowing pins 88 to be pulled.

Handle 18 also includes a rotatable ring 155 with internal teeth that are configured to engage with teeth on a gear 157 coupled to lead screw 134. Ring 155 is coupled to control knob 122 so that rotation of control knob 122 results in analogous motion of ring 155 and thus lead screw 134.

Handle 18 is generally configured for coordinated movement of multiple structures of system 10. For example, handle 18 is configured to allow a user to move outer sheath 12 (e.g., relative to inner catheter 14), move push-pull rods 84, and move pin release mandrel 92. Moreover, handle 18 is configured so that the appropriate structure can be moved at the appropriate time during the intervention so that implant 16 can be delivered in an efficient manner. Some examples of how the coordinated movement of system 10 may occur within handle 18 may be similar to those disclosed in U.S. Patent Application Pub. No. US 2010/0280495.

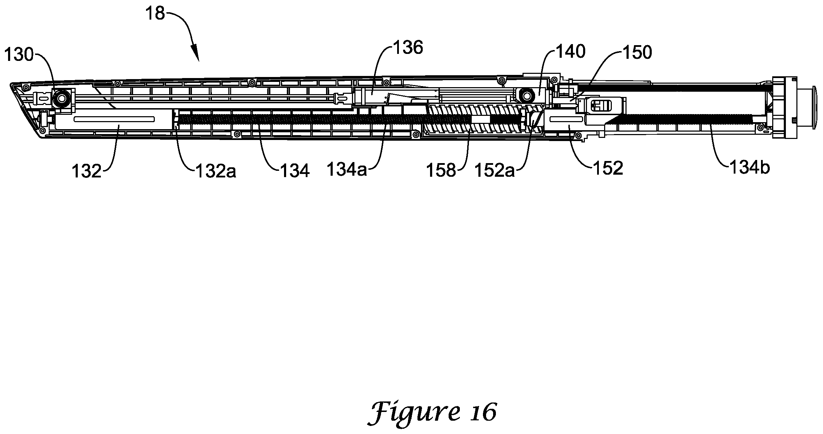

To help facilitate the coordinated movement, handle 18 may include a lost motion barrel 158. Lost motion barrel 158 is configured to engage carriages 132/152 and/or screws associated with carriages 132/152 at different times during the intervention to stop motion (e.g., create "lost motion" of the appropriate carriage). Figures 16-19 illustrate some of the coordinated motion achieved by handle 18. It should be noted that some elements of system 10 are not shown in Figures 16-20 for clarity. For example, Figure 16 illustrates a first position or state for handle 18 where outer sheath 12 is extended distally relative to inner catheter 14 (and handle 18) so as to fully sheath (e.g., contain) implant 16. While in this position, sheath carriage 132 is positioned adjacent to the distal end of handle 18. In addition, a rod screw 152a associated with push-pull rod carriage 152 is extended distally from push-pull rod carriage 152 and positioned within lost motion barrel 158. Upon rotation of control knob 122 (e.g., in the clockwise direction), lead screw 134 begins to rotate. Rotation of lead screw 134 causes sheath carriage 132 to move along lead screw 134 in the proximal direction, resulting in proximal movement of outer sheath 12 (e.g., "unsheathing" implant 16). This initial rotation of lead screw 134 also causes rod screw 152a to rotate. This may be because, for example, a knob or projection (not shown) on rod screw 152a may be engaged with a helical thread disposed along the interior of lost motion barrel 158. However, because rod screw 152a is spaced from push-pull rod carriage 152, it does not exert a force onto push-pull rod carriage 152. Thus, initial motion of control knob 122 does not result in movement of push-pull rod carriage 152 and, instead, only results in translation of sheath carriage 132 and rotation (and translation) of rod screw 152a.

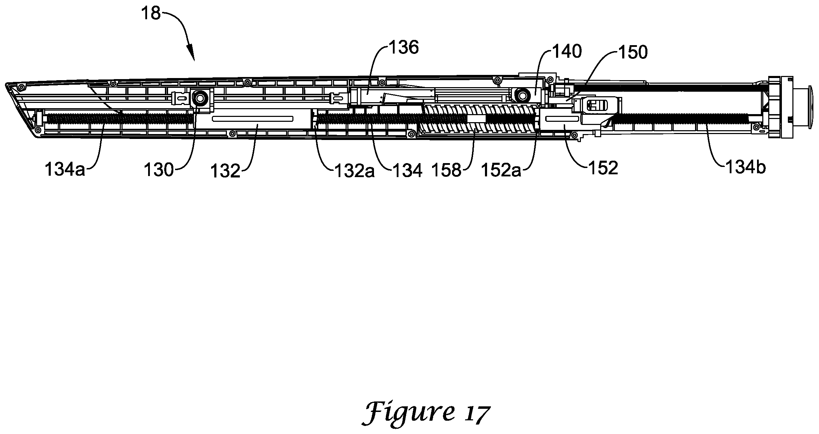

Eventually, rod screw 152a (e.g., the knob formed therein) reaches an essentially linear thread or pathway formed at the end of lost motion barrel 158. The linear thread allows rod screw 152a to translate along lead screw 134 to a position where rod screw 152a contacts (e.g., is threaded within and abuts) push-pull rod carriage 152. In doing so, rod screw 152a can contact and move proximally push-pull carriage 152. Accordingly, further rotation of lead screw 134 not only causes sheath carriage 132 to move proximally but also causes push-pull rod carriage 152 to move proximally as shown in Figure 17.

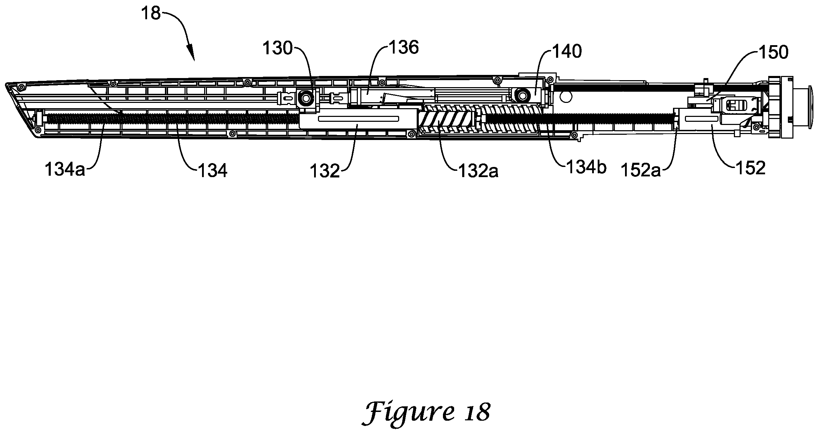

When sheath carriage 132 reaches lost motion barrel 158, a sheath carriage screw 132a of sheath carriage 132 enters lost motion barrel 158 as shown in Figure 18. This may occur in a manner similar to how rod screw 152a threads and unthreads with the helical thread formed along lost motion barrel 158. For example, while sheath carriage 132 is translating, sheath carriage screw 132a may follow an essentially linear thread or pathway formed along or adjacent to lost motion barrel 158. Upon reaching lost motion barrel 158, sheath carriage screw 132a (e.g., a knob or projection formed thereon) may shift into engagement with the helical thread within lost motion barrel 158 and rotate. This rotation "unthreads" sheath carriage screw 132a from sheath carriage 132. Accordingly, additional rotation of lead screw 134 results in continued proximal movement of push-pull rod carriage 152 while motion of sheath carriage 132 ceases.

In at least some embodiments, lead screw 134 has a plurality of portions, for example a first portion 134a and a second portion 134b, with a differing pitch to its thread. This may allow carriages 132/152 to travel at different rates along lead screw 134. For example, the pitch of lead screw 134 along which sheath carriage 132 translates may be generally more spaced or slanted than at positions adjacent to push-pull rod carriage 152. Accordingly, the coordinated movement of carriages 132/152 also may be configured so that sheath carriage 132 translates along lead screw 134 at a greater rate than push-pull rod carriage 152. Other configurations are contemplated where the above-mentioned configuration is reversed as well as further configurations where the pitch of lead screw 134 is essentially constant or includes a number of different pitch regions.

Sufficient proximal retraction of push-pull rod carriage 152, for example as shown in Figure 18, may result in push-pull rods 84 being sufficiently retracted so that posts 72 can engage and lock with buckles 76. When the clinician is satisfied that locking is complete (e.g., after verification via an appropriate visualization technique), the clinician may proximally retract pin release mandrel 92 in order to pull pins 88 from openings 98 and openings in push-pull rods 84 to release implant 16.

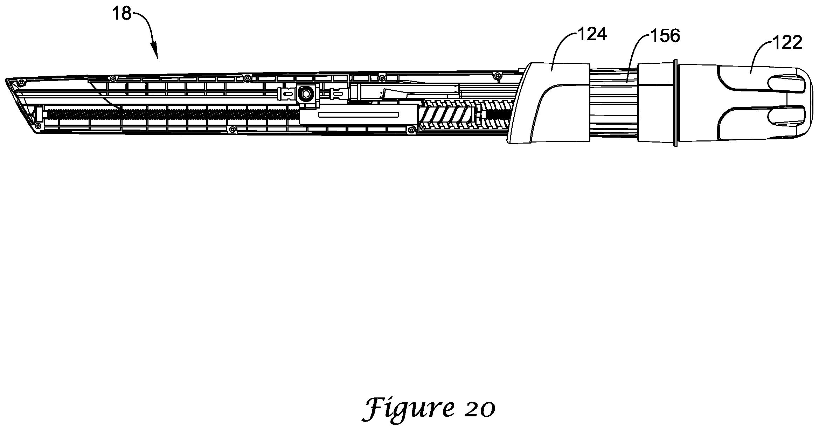

To initiate release of pins 88, door 124 may be slid distally along a collar 156 (which is positioned on handle 18) as shown in Figure 19. When door 124 is sufficiently advanced, door 124 and collar 156, together, can be rotated as shown in Figure 20. Push-pull rod carriage 152 may also include a radially-extending proximal flag member 164. In general, flag member 164 may be designed as a feature that can prevent collar 156 from being rotated earlier than desired (and, thus, prevent pins 88 from being pulled earlier than desired). For example, flag member 164 may be positioned within and follow a groove (not shown) along the interior of collar 156. While positioned within the groove, flag member 164 essentially forms a physical barrier that prevents collar 156 from rotating relative to handle housing 120. When push-pull rod carriage 152 is translated proximally to the back of handle housing 120 (e.g., when push-pull rods 84 are proximally retracted so as to lock posts 72 with buckles 76), flag member 164 exits the groove in collar 156. Accordingly, flag member 164 no longer impedes rotation of collar 156 and, as such, collar 156 can now be rotated to pull pins 88.

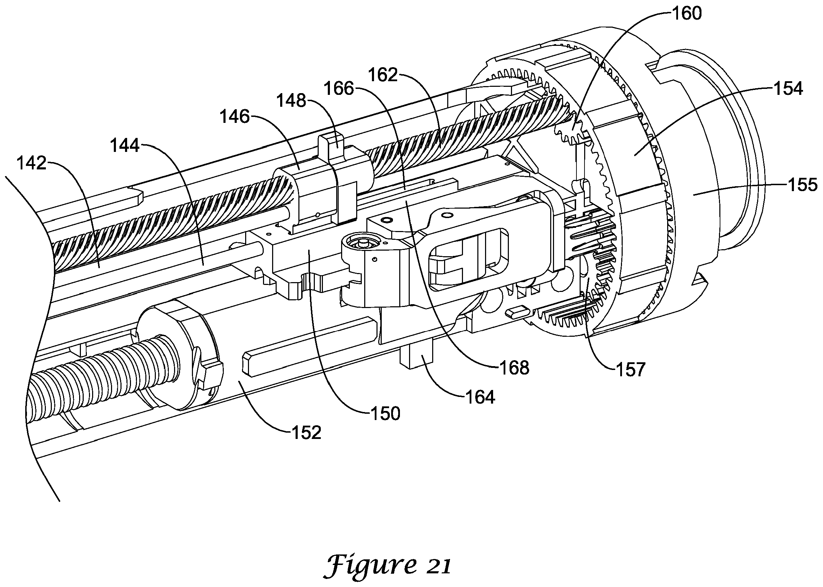

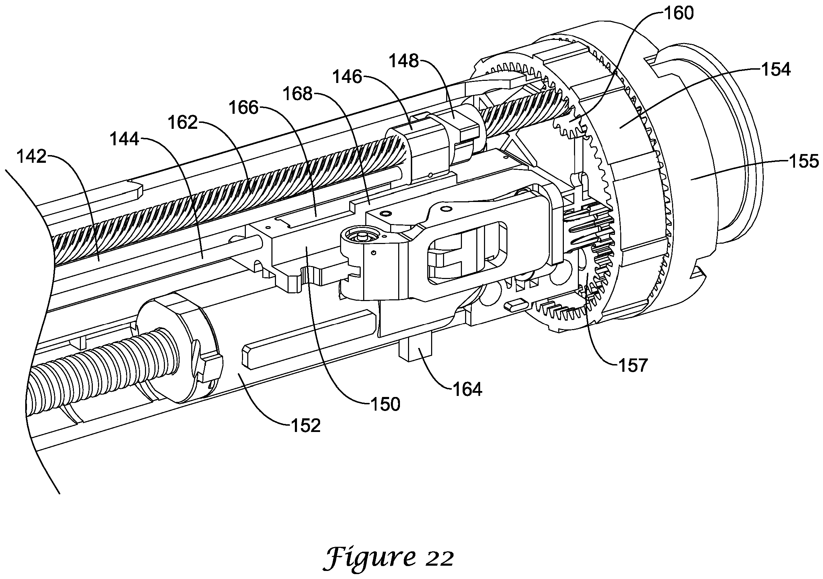

Collar 156, via ring 154, is associated with a gear 160 engaged with a secondary screw 162. Notches at a proximal end of collar 156 engage protrusions on ring 154 such that rotation of collar 156 causes corresponding rotation of ring 154 and thus secondary screw 162. The initial rotation of collar 156 is sufficient to rotate chock 148 (e.g., via a mechanical interaction between collar 156 and chock 148 that causes chock 148 to shift) from a first configuration where slider 146 (and, thus, pin release mandrel 92) is selectively locked to force limiter body 150, to a secondary configuration, which permits slider 146 to translate along secondary screw 162 as secondary screw 162 rotates, to proximally retract and pull pins 88 (e.g., via pin release mandrel 92). As seen in Figure 21, chock 148 in the first configuration engages a ridge 168 along a top portion of force limiter body 150 which forms a physical barrier that prevents proximal translation of slider 146 relative to force limiter body 150. When collar 156 is rotated to shift chock 148 into the secondary configuration, slider 146 can translate proximally within a groove 166 disposed in the top portion of force limiter body 150 (e.g., as seen in Figure 22), as collar 156 is rotated about the handle housing 120 to pull the pins 88 from the openings 98 and the openings in the distal ends of the push-pull rods 84. Once pins 88 have been removed, push-pull rods 84 may be withdrawn from implant 16, thereby deploying the implant at the target site (area of interest).

Following deployment of the implant 16, the control knob 122 may be rotated to move the sheath carriage 132 distally within the handle housing 120, thereby moving outer sheath 12 distally relative to inner catheter 14 and three-finger coupler 78 so as to cover or re-sheath the elements of system 10 disposed at the distal end. System 10 may then be removed from the patient's anatomy.

As can be seen in Figures 8-11, shifting implant 16 from a first or elongated configuration to a second or expanded configuration involves the proximal retraction of push-pull rods 84 so that posts 72 move proximally so as to engage and lock with buckles 76. In doing so, ridges 100 on posts 72 engage and lock with buckle 76. In order to properly lock with buckle 76, ridges 100 may need to be properly aligned or oriented (e.g., face the "correct" direction) so as to engage buckles 76. If ridges 100 do not engage buckles 76 while in the proper orientation, posts 72 may not lock with buckles 76 and implant 16 may not lock properly. For example, if one or more of posts 72 are twisted, post 72 may still be able to be seated within buckle 76, but ridge 100 would be oriented in an improper direction so that the post 72 could disassociate from buckle 76 and implant 16 may not properly remain in the expanded configuration. Furthermore, given that posts 72 may still be capable of passing into buckles 76 even when twisted, a clinician may believe under fluoroscopic visualization that implant 16 is locked when, in reality, one or more of ridges 100 may not be properly positioned within buckle 76 to effect proper locking of implant 16. Accordingly, a clinician may pull pins 88 believing that implant 16 is properly locked in the expanded configuration only to find out later that implant 16 is actually is not properly locked.

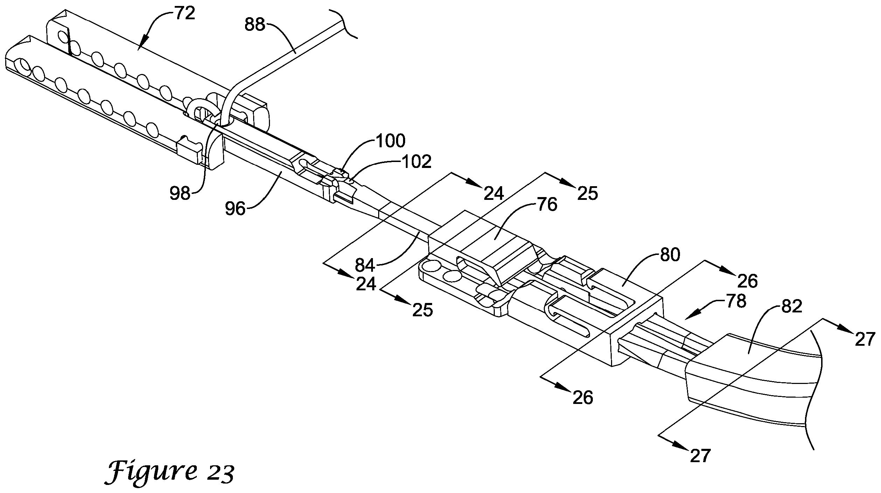

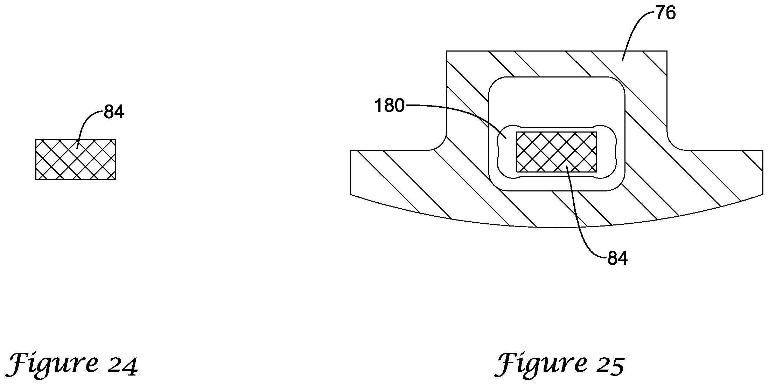

According to the invention, device 10 includes one or more features and/or structures that help maintain the proper alignment of posts 72 with buckles 76 so that locking integrity of implant 16 can be enhanced. In general, these features are aimed at maintaining proper alignment of posts 72 with buckles 76 and at reducing twisting of posts 72 and/or push-pull rods 84. As shown in Figures 23-24 push-pull rods 84 have at least a region where the outer surface thereof has a non-circular cross-sectional shape that is configured to engage with or "mate" with one or more of the structures associated with locking implant 16 (e.g., the "locking assembly", which may include buckle 76, collar 80, guide 82, inner catheter 14, and/or other structures of device 10). In this example, at least a portion of push-pull rod 84 may have a rectangular cross-sectional shape. Figure 25 illustrates that an interior passageway 180 of buckle 76 (e.g., where push-pull rod 84 may extend through) may have a shape corresponding to shape of push-pull rod 84. For example, at least a portion of the shape of passageway 180 corresponds to or otherwise resembles the rectangular shape of push-pull rod 84. This may allow push-pull rod to "key" or otherwise have a "lock and key" structural relationship with passageway 180. Accordingly, passageway 180 may prevent or otherwise limit any rotation of push-pull rod 84. Because of this, rotation of post 72 can be also be reduced and/or eliminated so that post 72 can properly engage buckle 76 to lock implant 16. In addition, in examples where only a portion of push-pull rod 84 has a non-circular cross-sectional shape, the shape of passageway 180 may also help to direct push-pull rod 84 therein and help to "correct" any rotation that may be present in push-pull rod 84.

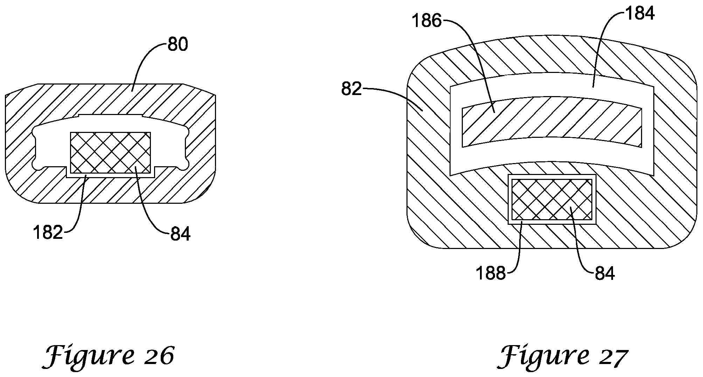

While the structure of buckle 76 may be relied upon to help maintain proper alignment of push-pull rod 84, other structures of system 10 may also be utilized. For example, Figure 26 illustrates that an interior passageway 182 of collar 80 may have a shape corresponding to shape of push-pull rod 84. Additionally, Figure 27 illustrates guide 82 that may include similar features. In Figure 27 it can be seen that guide 82 may include a first lumen 184 that is disposed about a finger 186 of coupler 78. Guide 82 may also include a second lumen 188 that may have a shape corresponding to shape of push-pull rod 84. Collectively, these figures illustrate that collar 80 and/or guide 82 may also be utilized to help maintain proper alignment of push-pull rod 84 so that implant 16 may be properly locked.

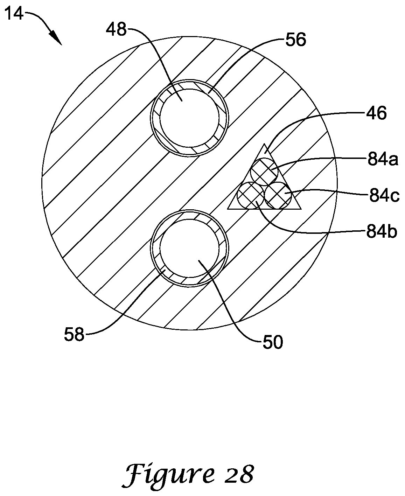

Figure 28 illustrates a cross-sectional view of inner catheter 14. Here it can be seen that first lumen 46 may have a non-circular cross-sectional shape. In this example, first lumen 46 may have a triangular cross-sectional shape. Accordingly, the three push-pull rods 84a/84b/84c (which, in this example are shown having a circular cross-sectional shape) extending therethrough may be confined within lumen 46 so that rotation of push-pull rods 84a/84b/84c relative to inner catheter 14 may be reduced if not altogether eliminated. Accordingly, in addition to the other components of system 10, inner catheter 14 may also be utilized to help maintain proper alignment of push-pull rod 84 so that implant 16 may be properly locked.

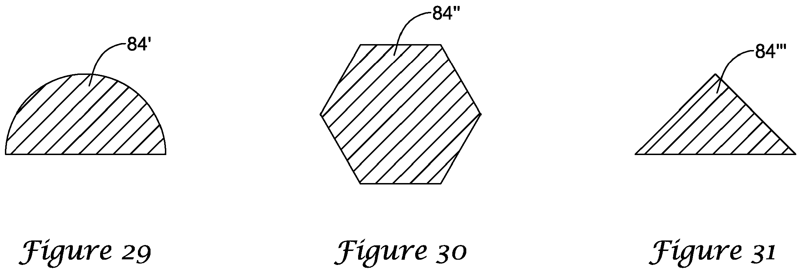

Figures 29-31 illustrate some of the additional cross-sectional shapes contemplated for push-pull rod 84. For example, Figure 29 illustrates push-pull rod 84' having a semi-circular or "D" cross-sectional shape. Figure 30 illustrates push-pull rod 84" having a hexagonal cross-sectional shape. Figure 31 illustrates push-pull rod 84" having a triangular cross-sectional shape. These are just examples as numerous other shapes are also contemplated including, for example, oval, semi-oval, polygonal, etc. Furthermore, as indicated above, the non-circular cross-sectional shape may be present along only a portion of the length of push-pull rods 84 or along substantially the entire length.

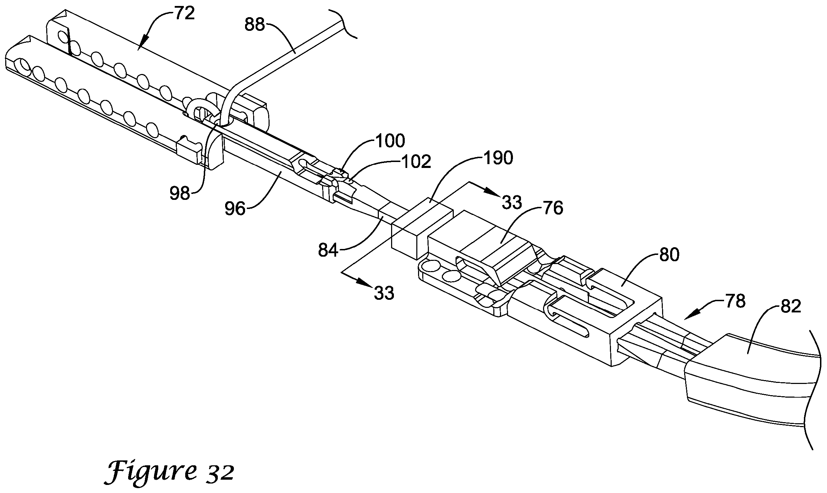

While the various components of system 10 may be configured to mate with push-pull rods 84, it is also contemplated that additional structures may also be utilized to reduce rotation of push-pull rods 84 and/or otherwise help maintain proper alignment of posts 72 with buckles 76. For example, Figure 32 illustrates a key member 190 that may be disposed about a portion of push-pull rod 84. In this example, key member 190 may be attached to buckle 76 (e.g., along a distal surface of buckle 76). However, this is not intended to be limiting as key member 190 may be positioned at other locations, as desired, along system 10. As shown in Figure 32, key member 190 may have an internal passageway 192, similar to other internal passageways disclosed herein, that may have a shape corresponding to shape of push-pull rod 84. Thus, the keyed relationship between push-pull rods 84 and key member 190 may help to reduce rotation of push-pull rods 84 and/or otherwise help maintain proper alignment of posts 72 with buckles 76.

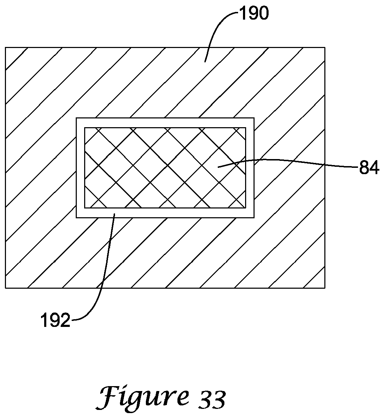

Figure 33 illustrates another example push-pull rod 194 that may be similar to other push-pull rods disclosed herein. Push-pull rod 194 may include a first portion 194a and a second portion 194b that are joined together with a swivel body 196. In some embodiments, the structural arrangement of push-pull rod 194 may form a "swivel" that allows portions of push-pull rod 194 to rotate. In the event that one of portions 194a/194b becomes rotated, the swivel may stop this rotation from being translated along the full length of push-pull rod 194. For example, if one of portions 194a/194b (e.g., a portion disposed on a proximal side of the swivel) becomes twisted, the swivel may help reduce the possibility that this twisting is transmitted further distally where it might otherwise cause twisting of post 72. In at least some embodiments, the swivel (e.g., swivel body 196) may be positioned adjacent to post 72 so that rotation of post 72 can be reduced. However, other locations may also be utilized.

The materials that can be used for the various components of system 10 (and/or other systems disclosed herein) and the various tubular members disclosed herein may include those commonly associated with medical devices. For simplicity purposes, the following discussion makes reference to outer sheath 12 and/or inner catheter 14. However, this is not intended to limit the devices and methods described herein, as the discussion may be applied to other similar tubular members and/or components of tubular members or devices disclosed herein.

Outer sheath 12 and/or inner catheter 14 may be made from a metal, metal alloy, polymer (some examples of which are disclosed below), a metal-polymer composite, ceramics, combinations thereof, and the like, or other suitable material. Some examples of suitable metals and metal alloys include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL® 625, UNS: N06022 such as HASTELLOY® C-22®, UNS: N10276 such as HASTELLOY® C276®, other HASTELLOY® alloys, and the like), nickel-copper alloys (e.g., UNS: N04400 such as MONEL® 400, NICKEL VAC® 400, NICORROS® 400, and the like), nickel-cobalt-chromium-molybdenum alloys (e.g., UNS: R30035 such as MP35-N® and the like), nickel-molybdenum alloys (e.g., UNS: N10665 such as HASTELLOY® ALLOY B2®), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY®, PHYNOX®, and the like); platinum enriched stainless steel; titanium; combinations thereof; and the like; or any other suitable material.

As alluded to herein, within the family of commercially available nickel-titanium or nitinol alloys, is a category designated "linear elastic" or "non-super-elastic" which, although may be similar in chemistry to conventional shape memory and super elastic varieties, may exhibit distinct and useful mechanical properties. Linear elastic and/or non-super-elastic nitinol may be distinguished from super elastic nitinol in that the linear elastic and/or non-super-elastic nitinol does not display a substantial "superelastic plateau" or "flag region" in its stress/strain curve like super elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear that the super elastic plateau and/or flag region that may be seen with super elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super elastic nitinol in that linear elastic and/or non-super-elastic nitinol may accept up to about 2-5% strain while remaining substantially elastic (e.g., before plastically deforming) whereas super elastic nitinol may accept up to about 8% strain before plastically deforming. Both of these materials can be distinguished from other linear elastic materials such as stainless steel (that can also can be distinguished based on its composition), which may accept only about 0.2 to 0.44 percent strain before plastically deforming.

In some embodiments, the linear elastic and/or non-super-elastic nickel-titanium alloy is an alloy that does not show any martensite/austenite phase changes that are detectable by differential scanning calorimetry (DSC) and dynamic metal thermal analysis (DMTA) analysis over a large temperature range. For example, in some embodiments, there may be no martensite/austenite phase changes detectable by DSC and DMTA analysis in the range of about -60 degrees Celsius (°C) to about 120 °C in the linear elastic and/or non-super-elastic nickel-titanium alloy. The mechanical bending properties of such material may therefore be generally inert to the effect of temperature over this very broad range of temperature. In some embodiments, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. In other words, across a broad temperature range, the linear elastic and/or non-super-elastic nickel-titanium alloy maintains its linear elastic and/or non-super-elastic characteristics and/or properties.

In some embodiments, the linear elastic and/or non-super-elastic nickel-titanium alloy may be in the range of about 50 to about 60 weight percent nickel, with the remainder being essentially titanium. In some embodiments, the composition is in the range of about 54 to about 57 weight percent nickel. One example of a suitable nickel-titanium alloy is FHP-NT alloy commercially available from Furukawa Techno Material Co. of Kanagawa, Japan. Some examples of nickel titanium alloys are disclosed in U.S. Patent Nos. 5,238,004 and 6,508,803. Other suitable materials may include ULTANIUM™ (available from Neo-Metrics) and GUM METAL™ (available from Toyota). In some other embodiments, a superelastic alloy, for example a superelastic nitinol can be used to achieve desired properties.

In at least some embodiments, portions or all of outer sheath 12 and inner catheter 14 may also be doped with, made of, or otherwise include a radiopaque material. Radiopaque materials are understood to be materials capable of producing a relatively bright image on a fluoroscopy screen or another imaging technique during a medical procedure. This relatively bright image aids the user of system 10 in determining its location. Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of system 10 to achieve the same result.

In some embodiments, a degree of Magnetic Resonance Imaging (MRI) compatibility is imparted into system 10. For example, outer sheath 12 and inner catheter 14, or portions thereof, may be made of a material that does not substantially distort the image and create substantial artifacts (i.e., gaps in the image). Certain ferromagnetic materials, for example, may not be suitable because they may create artifacts in an MRI image. Outer sheath 12 and inner catheter 14, or portions thereof, may also be made from a material that the MRI machine can image. Some materials that exhibit these characteristics include, for example, tungsten, cobalt-chromium-molybdenum alloys (e.g., UNS: R30003 such as ELGILOY®, PHYNOX®, and the like), nickel-cobalt-chromium-molybdenum alloys (e.g., UNS: R30035 such as MP35-N® and the like), nitinol, and the like, and others.

A sheath or covering (not shown) may be disposed over portions or all of outer sheath 12 and inner catheter 14 that may define a generally smooth outer surface for system 10. In other embodiments, however, such a sheath or covering may be absent from a portion of all of system 10, such that outer sheath 12 and inner catheter 14 may form an outer surface. The sheath may be made from a polymer or other suitable material. Some examples of suitable polymers may include polytetrafluoroethylene (PTFE), ethylene tetrafluoroethylene (ETFE), fluorinated ethylene propylene (FEP), polyoxymethylene (POM, for example, DELRIN® available from DuPont), polyether block ester, polyurethane (for example, Polyurethane 85A), polypropylene (PP), polyvinylchloride (PVC), polyether-ester (for example, ARNITEL® available from DSM Engineering Plastics), ether or ester based copolymers (for example, butylene/poly(alkylene ether) phthalate and/or other polyester elastomers such as HYTREL® available from DuPont), polyamide (for example, DURETHAN® available from Bayer or CRISTAMID® available from Elf Atochem), elastomeric polyamides, block polyamide/ethers, polyether block amide (PEBA, for example available under the trade name PEBAX®), ethylene vinyl acetate copolymers (EVA), silicones, polyethylene (PE), Marlex high-density polyethylene, Marlex low-density polyethylene, linear low density polyethylene (for example REXELL®), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR®), polysulfone, nylon, nylon-12 (such as GRILAMID® available from EMS American Grilon), perfluoro(propyl vinyl ether) (PFA), ethylene vinyl alcohol, polyolefin, polystyrene, epoxy, polyvinylidene chloride (PVdC), poly(styrene-b-isobutylene-b-styrene) (for example, SIBS and/or SIBS 50A), polycarbonates, ionomers, biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6 percent LCP.

In some embodiments, the exterior surface of the system 10 (including, for example, the exterior surface of outer sheath 12 and inner catheter 14) may be sandblasted, beadblasted, sodium bicarbonate-blasted, electropolished, etc. In these as well as in some other embodiments, a coating, for example a lubricious, a hydrophilic, a protective, or other type of coating may be applied over portions or all of the sheath, or in embodiments without a sheath over portion of outer sheath 12 and inner catheter 14, or other portions of system 10. Alternatively, the sheath may comprise a lubricious, hydrophilic, protective, or other type of coating. Hydrophobic coatings such as fluoropolymers provide a dry lubricity which improves device handling and device exchanges. Lubricious coatings improve steerability and improve lesion crossing capability. Suitable lubricious polymers are well known in the art and may include silicone and the like, hydrophilic polymers such as high-density polyethylene (HDPE), polytetrafluoroethylene (PTFE), polyarylene oxides, polyvinylpyrolidones, polyvinylalcohols, hydroxy alkyl cellulosics, algins, saccharides, caprolactones, and the like, and mixtures and combinations thereof. Hydrophilic polymers may be blended among themselves or with formulated amounts of water insoluble compounds (including some polymers) to yield coatings with suitable lubricity, bonding, and solubility. Some other examples of such coatings and materials and methods used to create such coatings can be found in U.S. Patent Nos. 6,139,510 and 5,772,609.

The coating and/or sheath may be formed, for example, by coating, extrusion, co-extrusion, interrupted layer co-extrusion (ILC), or fusing several segments end-to-end. The layer may have a uniform stiffness or a gradual reduction in stiffness from the proximal end to the distal end thereof. The gradual reduction in stiffness may be continuous as by ILC or may be stepped as by fusing together separate extruded tubular segments. The outer layer may be impregnated with a radiopaque filler material to facilitate radiographic visualization. Those skilled in the art will recognize that these materials can vary widely without deviating from the scope of the present invention.

It should be understood that this disclosure is, in many respects, only illustrative. Changes may be made in details, particularly in matters of shape, size, and arrangement of steps without exceeding the scope of the invention. This may include, to the extent that it is appropriate, the use of any of the features of one example embodiment being used in other embodiments. The invention's scope is, of course, defined in the language in which the appended claims are expressed.

## Claims

1. A medical device delivery system, comprising:
an outer sheath (12);
an inner catheter (14) disposed within the outer sheath (12);
an implant (16) releasably coupled to the inner catheter (14);
wherein the implant (16) is configured to shift between a first elongated configuration and a second expanded configuration, the implant comprising a post (72) and a buckle (76) configured to lock the implant (16) in the second expanded configuration, wherein the post (72) and the buckle (76) are attached to the implant (16);
a push-pull rod (84) for shifting the implant (16) between the first configuration and the second, the push-pull rod (84) being coupled to the inner catheter (14) and configured to pull the post (72) into engagement with the buckle (76);
**characterized in that**
a locking assembly is disposed about the push-pull rod (84), the locking assembly having an interior passageway with a non-circular cross-sectional shape, the interior passageway of the locking assembly being defined
- within the buckle (76) coupled to the implant (16), or
- within a collar (80) coupled to the inner catheter (14), or
- within a guide (82) coupled to the inner catheter (14), or
- by a non-circular lumen (46) formed within the inner catheter (14);
wherein at least a portion of an outer surface of the push-pull rod (84) has a non-circular cross-sectional shape; and
wherein the interior passageway of the locking assembly with the non-circular cross-sectional shape corresponds to the non-circular cross-sectional shape of the push-pull rod (84), thereby reducing twisting of the push-pull rod (84).

2. The system of claim 1, wherein the portion of the outer surface of the push-pull rod (84) having the non-circular cross-sectional shape has a rectangular shape.

3. The system of any one of claims 1-2, wherein the portion of the outer surface of the push-pull rod (84) having the non-circular cross-sectional shape has a semi-circular shape.

4. The system of any one of claims 1-3, wherein the portion of the outer surface of the push-pull rod (84) having the non-circular cross-sectional shape has a polygonal shape.

5. The system of any one of claims 1-4, wherein the portion of the outer surface of the push-pull rod (84) having the non-circular cross-sectional shape has a triangular shape.

6. The system of any one of claims 1-5, wherein the interior passageway of the locking assembly with the non-circular cross-sectional shape corresponding to the non-circular cross-sectional shape of the push-pull rod (84) is defined within a key member (190) disposed adjacent to a buckle (76), the buckle (76) being coupled to the implant (16).

7. The system of any one of claims 1-6, wherein the push-pull rod (194) includes a first region (194a), a second region (194b), and a swivel body (196) coupling the first region (194a) with the second region (194b).

## Patentansprüche

1. System zum Zuführen einer medizinischen Vorrichtung, umfassend:
eine äußere Hülse (12),
einen in der äußeren Hülse (12) angeordneten inneren Katheter (14),
ein freigebbar an den inneren Katheter (14) gekoppeltes Implantat (16),
wobei das Implantat (16) dazu ausgestaltet ist, sich zwischen einer ersten länglichen Konfiguration und einer zweiten expandierten Konfiguration zu verschieben, wobei das Implantat einen Pfosten (72) und eine Spange (76) umfasst, die dazu ausgestaltet sind, das Implantat (16) in der zweiten expandierten Konfiguration zu arretieren, wobei der Pfosten (72) und die Spange (76) an dem Implantat (16) angebracht sind,
eine Zug/Druckstange (84) zum Verschieben des Implantats (16) zwischen der ersten Konfiguration und der zweiten, wobei die Zug/Druckstange (84) an den inneren Katheter (14) gekoppelt und dazu ausgestaltet ist, den Pfosten (72) in Eingriff mit der Spange (76) zu ziehen,
**dadurch gekennzeichnet, dass**
eine Arretieranordnung um die Zug/Druckstange (84) herum angeordnet ist, wobei die Arretieranordnung einen inneren Durchgang mit einer nicht kreisförmigen Querschnittsform hat, wobei der innere Durchgang der Arretieranordnung wie folgt definiert ist:
- in der Spange (76), die an das Implantat (16) gekoppelt ist, oder
- in einem Bund (80), der an den inneren Katheter (14) gekoppelt ist, oder
- in einer Führung (82), die an den inneren Katheter (14) gekoppelt ist, oder
- durch ein in dem inneren Katheter (14) gebildetes nicht kreisförmiges Lumen (46),
wobei mindestens ein Abschnitt einer Außenfläche der Zug/Druckstange (84) eine nicht kreisförmige Querschnittsform hat und
wobei der innere Durchgang der Arretieranordnung mit der nicht kreisförmigen Querschnittsform der nicht kreisförmigen Querschnittsform der Zug/Druckstange (84) entspricht, wodurch ein Verdrehen der Zug/Druckstange (84) reduziert wird.

2. System nach Anspruch 1, wobei der Abschnitt der Außenfläche der Zug/Druckstange (84), der die nicht kreisförmige Querschnittsform hat, eine rechteckige Form hat.

3. System nach einem der Ansprüche 1 - 2, wobei der Abschnitt der Außenfläche der Zug/Druckstange (84), der die nicht kreisförmige Querschnittsform hat, eine halbkreisförmige Form hat.

4. System nach einem der Ansprüche 1 - 3, wobei der Abschnitt der Außenfläche der Zug/Druckstange (84), der die nicht kreisförmige Querschnittsform hat, eine polygone Form hat.

5. System nach einem der Ansprüche 1 - 4, wobei der Abschnitt der Außenfläche der Zug/Druckstange (84), der die nicht kreisförmige Querschnittsform hat, eine dreieckige Form hat.

6. System nach einem der Ansprüche 1 - 5, wobei der innere Durchgang der Arretieranordnung mit der nicht kreisförmigen Querschnittsform, die der nicht kreisförmigen Querschnittsform der Zug/Druckstange (84) entspricht, in einem Schlüsselglied (190) definiert ist, das einer Spange (76) benachbart angeordnet ist, wobei die Spange (76) an das Implantat (16) gekoppelt ist.

7. System nach einem der Ansprüche 1 - 6, wobei die Zug/Druckstange (194) einen ersten Bereich (194a), einen zweiten Bereich (194b) und einen Schwenkkörper (196) aufweist, der den ersten Bereich (194a) an den zweiten Bereich (194b) koppelt.

## Revendications

1. Système de pose de dispositif médical, comprenant :
une gaine externe (12) ;
un cathéter interne (14) disposé à l'intérieur de la gaine externe (12) ;
un implant (16) accouplé de manière libérable au cathéter interne (14) ;
dans lequel l'implant (16) est configuré pour se décaler entre une première configuration allongée et une seconde configuration déployée, l'implant comprenant un montant (72) et une boucle (76) configurés pour verrouiller l'implant (16) dans la seconde configuration déployée,
dans laquelle le montant (72) et la boucle (76) sont fixés à l'implant (16) ;
une tige de poussée-traction (84) pour décaler l'implant (16) entre la première configuration et
la seconde, la tige de poussée-traction (84) étant accouplée au cathéter interne (14) et
configurée pour tirer le montant (72) vers une entrée en prise avec la boucle (76) ;
**caractérisé en ce que**
un ensemble de verrouillage est disposé autour de la tige de poussée-traction (84), l'ensemble de verrouillage possédant un passage intérieur ayant une forme de coupe transversale non circulaire, le passage intérieur de l'ensemble de verrouillage étant défini
- à l'intérieur de la boucle (76) accouplée à l'implant (16), ou
- à l'intérieur d'un collier (80) accouplé au cathéter interne (14), ou
- à l'intérieur d'un guide (82) accouplé au cathéter interne (14), ou
- par une lumière non circulaire (46) formée à l'intérieur du cathéter interne (14) ;
dans lequel au moins une partie d'une surface externe de la tige de poussée-traction (84) a une forme de coupe transversale non circulaire ; et
dans lequel le passage intérieur de l'ensemble de verrouillage avec la forme de coupe transversale non circulaire correspond à la forme de coupe transversale non circulaire de la tige de poussée-traction (84), ce qui permet de réduire une torsion de la tige de poussée-traction (84).

2. Système selon la revendication 1, dans lequel la partie de la surface externe de la tige de poussée-traction (84) ayant la forme de coupe transversale non circulaire a une forme rectangulaire.

3. Système selon l'une quelconque des revendications 1 et 2, dans lequel la partie de la surface externe de la tige de poussée-traction (84) ayant la forme de coupe transversale non circulaire a une forme semi-circulaire.

4. Système selon l'une quelconque des revendications 1 à 3, dans lequel la partie de la surface externe de la tige de poussée-traction (84) ayant la forme de coupe transversale non circulaire a une forme polygonale.

5. Système selon l'une quelconque des revendications 1 à 4, dans lequel la partie de la surface externe de la tige de poussée-traction (84) ayant la forme de coupe transversale non circulaire a une forme triangulaire.

6. Système selon l'une quelconque des revendications 1 à 5, dans lequel le passage intérieur de l'ensemble de verrouillage avec la forme de coupe transversale non circulaire correspondant à la forme de coupe transversale non circulaire de la tige de poussée-traction (84) est défini à l'intérieur d'un élément clavette (190) disposé de façon adjacente à une boucle (76), la boucle (76) étant accouplée à l'implant (16).

7. Système selon l'une quelconque des revendications 1 à 6, dans lequel la tige de poussée-traction (194) comprend une première région (194a), une seconde région (194b) et un corps de tourillon (196) accouplant la première région (194a) à la seconde région (194b).
